(19) **Europäisches Patentamt**

**European Patent Office**

**Office européen des brevets**

(11) **EP 0 576 828 B2**

(12) # NEUE EUROPÄISCHE PATENTSCHRIFT

(45) Veröffentlichungstag und Bekanntmachung des Hinweises auf die Entscheidung über den Einspruch:
**19.04.2000 Patentblatt 2000/16**

(45) Hinweis auf die Patenterteilung:
**23.04.1997 Patentblatt 1997/17**

(21) Anmeldenummer: **93108470.1**

(22) Anmeldetag: **26.05.1993**

(51) Int. Cl.7: **B01J 23/40**, B01J 35/02

(54) **Katalysator und Verfahren zur selektiven Hydrierung ungesättigter Verbindungen sowie Verfahren zur Herstellung des Katalysators**

Catalyst and unsatured compound hydrogenation process and also a process for the preparation of the catalyst

Catalyseur et procédé d'hydrogénation sélective de composés insaturés ainsi qu'un procédé de préparation du catalyseur

(84) Benannte Vertragsstaaten:
**BE DE ES FR IT NL**

(30) Priorität: **27.06.1992 DE 4221139**

(43) Veröffentlichungstag der Anmeldung:
**05.01.1994 Patentblatt 1994/01**

(73) Patentinhaber:
**Degussa-Hüls Aktiengesellschaft
60287 Frankfurt am Main (DE)**

(72) Erfinder:
- **Lüken, Hans-Gerd, Dr.
  D-4370 Marl (DE)**
- **Fischer, Lothar, Dr.
  D-4370 Marl (DE)**
- **Droste, Wilhelm ,Dr.
  D-4370 Marl (DE)**
- **Nowitzki, Bernd, Dr.
  D-4370 Marl (DE)**

(74) Vertreter:
**Patentanwälte
Sternagel & Fleischer
Braunsberger Feld 29
51429 Bergisch Gladbach (DE)**

(56) Entgegenhaltungen:
WO-A-90/08592          DE-A- 2 758 274
DE-A- 2 758 318        GB-A- 2 020 993
US-A- 4 966 878

- **Ullmans Enzyklopädie der techn. Chemie, 4.Auflage Band 7 (1974) 296-299**
- **Physical and Chemical Aspects of Adsorbents and Catalysts (1970), 185, 187, 189, 196, 200**
- **Ullmans Enzyklopädie der techn. Chemie, 4. Auflage Band 13 (1977), 136, 139, 519, 546**
- **Girdler Catalyst G-55A for selective hydrogenation, Aug. 1991 mit Qnlagen D8a und D8b**
- **Girdler Catalyst G-58B for selective hydrogenation, Aug. 1991 mit Anlagen D9a und D9b**
- **Girdler Catalyst G-74 for gas purification, Sept. 1977 mit Anlagen D10a und D10B**
- **Proc. Sixth Int.Congress. Catalysis, G.C.Bond et al (Ed.) 150, 151**

EP 0 576 828 B2

**Beschreibung**

Die Erfindung betrifft einen Katalysator zur selektiven Hydrierung ungesättigter Verbindungen auf Basis von Edelmetall und/oder Edelmetalloxid auf einem Aluminiumoxidträger sowie ein Verfahren zur Herstellung des Katalysators.

Des weiteren betrifft die Erfindung ein Verfahren zur selektiven Hydrierung ungesättigter Verbindungen.

Selektive Hydrierungen an Katalysatoren sind in der chemischen Industrie und in der ölverarbeitenden Industrie wohlbekannt.

Anwendungen von selektiven Hydrierungen können hierbei verschiedenen Zwecken dienen.

So kann beispielsweise eine selektive Hydrierung betrieben werden, um bestimmte Produkte. wie z. B. Ethen oder Propen, von unerwünschten Nebenprodukten. wie Diolefinen und Acetylenen, die eine weitere Verarbeitung der Olefin stören würden, zu befreien.

Ein anderer Anwendungsfall kann die selektive Hydrierung von Substanzen sein, die sich im Verlaufe einer Reaktion bilden und als unerwünschte Nebenprodukte eine Verringerung der Gesamtausbeute von Zielprodukten mit sich bringen. Durch selektive Hydrierung lassen sich solche Nebenprodukte ggf. wieder in Ausgangssubstanzen überführen, die dann wieder in den Prozeß zurückgeschleust werden können. Hierzu ist ein typisches Beispiel die selektive Hydrierung von $\alpha$-Methylstyrol zu Cumol in Prozessen zur Herstellung von Phenol und Aceton, z. B. bei der Hockschen Phenolsynthese (siehe Weissermel/Arpe, Industrielle Organische Chemie, Verlag Chemie 1976, S. 291 ff). Als Nebenprodukt der Phenolsynthese fällt $\alpha$-Methylstyrol an. Durch selektive Hydrierung des $\alpha$-Methylstyrols zu Cumol wird erneut Ausgangssubstanz erhalten, welche durch Zurückschleusen in den Prozeß letztendlich zu einer verbesserten Wirtschaftlichkeit des Prozesses führt.

Wesentlich für eine selektive Hydrierung ist u. a. die Anfangsselektivität des eingesetzten Katalysators. Falls zu Beginn der selektiven Hydrierung zuviel Nebenprodukte, z. B. durch unerwünschte Totalhydrierung, gebildet werden, ergeben sich neben Ausbeuteverlusten auch langwierige Anfahrprozeduren der Hydrieranlage, was ebenfalls zu Einbußen der Wirtschaftlichkeit des Verfahrens führt.

So offenbart DE-OS 27 58 318 ein Verfahren zur selektiven Hydrierung von ungesättigten Kohlenwasserstoffen, wobei der Hydrierungskatalysator zur Steigerung seiner Selektivität eine angemessene Zeit mit u. a. den zu hydrierenden Komponenten in Abwesenheit von Wasserstoff in Berührung gebracht wird. DE-OS 27 58 274 lehrt, daß die Hydrierungsselektivität eines Hydrierungskatalysators dadurch gesteigert werden kann, daß der Hydrierungskatalysator eine ausreichende Zeit lang mit gasförmigem Ammoniak behandelt wird und anschließend die selektive katalytische Hydrierung der ungesättigten Verbindungen mit Wasserstoff durchgeführt wird.

Als Katalysatoren werden insbesondere Edelmetalle. wie Ruthenium, Rhodium, Palladium oder Platin, auf Trägern verwendet. Geeignete Träger sind z. B. Tone. Magnesiumoxid. Gele von Siliziumdioxid und Aluminiumoxid, Zeolithe, Aktivkohle und aktivierte Aluminiumoxide, die jeweils in verschiedenen Formen, wie Pellets, Kugeln etc., vorliegen können.

Beide Verfahren gemäß dem Stand der Technik sind aufwendig und führen durch die für den Katalysator notwendigen Anfahrprozeduren zu erhöhten Betriebskosten.

Der Erfindung liegt die Aufgabe zugrunde, einen Katalysator zu entwickeln, der eine hohe Anfangsselektivität aufweist und somit einen sofortigen vollen Einsatz in Prozessen zur selektiven Hydrierung von ungesättigten Verbindungen ermöglicht.

Es wurde nun überraschenderweise gefunden, daß ein Katalysator auf Basis von Edelmetall und/oder Edelmetalloxid auf einem Aluminiumoxidträger eine sehr hohe Anfangsselektivität besitzt und somit sofort voll zur selektiven ven Hydrierung von ungesättigten Verbindungen eingesetzt werden kann, wenn der Frischkatalysator das folgende Röntgenbeugungsmuster aufweist:

| d $[10^{-10}m]$ | I/Io |
|---|---|
| 4,51 | 5 - 10 |
| 2,76 | } 15 - 40 |
| 2,64 | |
| 2,37 | 40 - 45 |
| 2,27 | 25 - 35 |
| 2,15 | bis 15 |
| 1,97 | 55 - 70 |
| 1,66 | bis 15 |
| 1,52 | 15 - 30 |
| 1,39 | 100 |
| 1,31 | bis 15 |

Mit Hilfe des erfindungsgemäßen Katalysators lassen sich langwierige und kostenintensive Anfahrprozeduren vermeiden.

Gegenstand der vorliegenden Erfindung ist daher ein Katalysator zur selektiven Hydrierung ungesättigter Verbindungen auf Basis von Edelmetall und/oder Edelmetalloxid auf einem Aluminiumoxidträger, der dadurch gekennzeichnet ist, daß der Frischkatalysator das folgende Röntgenbeugungsmuster aufweist:

| d $[10^{-10}m]$ | I/Io |
|---|---|
| 4,51 | 5 - 10 |
| 2,76 | } 15 - 40 |
| 2,64 | |
| 2,37 | 40 - 45 |
| 2,27 | 25 - 35 |
| 2,15 | bis 15 |
| 1,97 | 55 - 70 |
| 1,66 | bis 15 |
| 1,52 | 15 - 30 |
| 1,39 | 100 |
| 1,31 | bis 15 |

Hierbei bedeuten d der Netzebenenabstand und I/Io die relativen Intensitäten der Röntgenreflexe. Des weiteren ist Gegenstand der vorliegenden Erfindung ein Verfahren zur Herstellung des Katalysators gemäß den Ansprüchen 1 bis 14 das dadurch gekennzeichnet ist, daß das Edelmetall in Form einer wäßrigen Edelmetallsalzlösung in den Aluminiumoxidträger eingebracht wird.

Vorzugsweise wird der mit einer wäßrigen Edelmetallsalzlösung behandelte Aluminiumoxidträger bei einer Temperatur von 50 bis 200 °C getrocknet und anschließend bei einer Temperatur von 250 bis 650 °C thermisch nachbehandelt.

Außerdem ist Gegenstand der vorliegenden Erfindung ein Verfahren zur selektiven Hydrierung ungesättigter Verbindungen. das dadurch gekennzeichnet ist, daß ein Katalysator gemäß den Ansprüchen 1 bis 11 eingesetzt wird.

Der erfindungsgemäße Katalysator enthält vorzugsweise Palladium und/oder Palladiumoxid der Formel $PdO_x$ mit x = 0 bis 1.

Darüber hinaus kann der Katalysator neben Palladium und/oder Palladiumoxid Edelmetalle und/oder Edelmetalloxide der Gruppe Platin, Iridium, Ruthenium und Rhodium enthalten.

Prinzipiell sind für den erfindungsgemäßen Katalysator alle Edelmetalle geeignet, die üblicherweise in Hydrierungskatalysatoren verwendet werden.

Vorzugsweise beträgt der Gehalt des erfindungsgemäßen Katalysators an Edelmetallen und/oder Edelmetalloxiden insgesamt 0,01 bis 3 Gew.-%. bezogen auf den Katalysator, besonders vorzugsweise insgesamt 0,1 bis 1 Gew.-%, bezogen auf den Katalysator.

Die Edelmetalle und/oder Edelmetalloxide befinden sich vorzugsweise in einer äußeren Randzone des erfindungsgemäßen Katalysators im Bereich von 5 bis 200 μm, besonders vorzugsweise im Bereich von 30 bis 100 μm.

Für den erfindungsgemäßen Katalysator sind verschiedene Aluminiumoxidphasen als Trägermaterialien geeignet.

Die im Frischkatalysator enthaltenen Primärkristallite weisen bevorzugt eine Größe von 0,1 bis 30 nm, besonders bevorzugt eine Größe von 1 bis 15 nm auf. Der Natriumgehalt des Frischkatalysators kann 0,001 bis 3 Gew.-%. vorzugsweise 0,01 bis 1 Gew.-%. betragen.

Darüber hinaus können im Frischkatalysator andere Alkali- und/oder Erdalkaliverbindungen, Nitrate, Carbonate, Phosphate, Sulfate, Siliziumoxide und Eisenoxide mit jeweils einem Gehalt von ≤ Gew.-% enthalten sein.

Die BET-Gesamtoberflache des Frischkatalysators kann zwischen 50 und 250 $m^2$/g Frischkatalysator, insbesondere zwischen 100 bis 180 $m^2$/g Frischkatalysator, liegen. Das Porenvolumen des Frischkatalysators kann 0,2 bis 0,8 ml/g Frischkatalysator, insbesondere 0,3 bis 0,6 ml/g Frischkatalysator, und die Porengröße des Frischkatalysators im wesentlichen 3 bis 30 nm, insbesondere 4 bis 20 nm, betragen.

Unter Frischkatalysator ist der erfindungsgemäße Katalysator vor Einsatz zur selektiven Hydrierung zu verstehen.

Die selektive Hydrierung ungesättigter Verbindungen wird mit Hilfe des erfindungsgemäßen Katalysators vorzugsweise bei Drücken von 2 bis 30 bar abs. und bei Temperaturen von 40 bis 180 °C durchgeführt. Geeigneterweise stellt man ein Molverhältnis von Wasserstoff zu ungesättigten Verbindungen von 1 : 1 bis 3 : 1 ein.

Das erfindungsgemäße Verfahren zur selektiven Hydrierung ist insbesondere für α-Methylstyrol als ungesättigte Verbindung geeignet.

Bei der Herstellung des erfindungsgemäßen Katalysators können als Aluminiumoxidvorläuferverbindungen für den Aluminiumoxidträger z. B. Bayerit und/oder Pseudo-Boehmit verwendet werden. Die Aluminiumoxidvorläuferverbindungen können mit einer Säure, vorzugsweise mit Salpetersäure, behandelt und anschließend verformt werden. Die so erhaltenen Formkörper können dann z. B. bei Temperaturen von 80 bis 140 °C getrocknet und bei Temperaturen von 400 bis 700 °C kalziniert werden.

Ein so hergestellter Aluminiumoxidträger kann dann mit einer wäßrigen Edelmetallsalzlösung imprägniert werden. Die Imprägnierung kann bei Temperaturen des Aluminiumoxidträgers von Raumtemperatur bis 100 °C erfolgen. Der imprägnierte Aluminiumoxidträger wird dann vorzugsweise bei einer Temperatur von 50 bis 200 °C getrocknet und anschließend bei einer Temperatur von 250 bis 650 °C thermisch nachbehandelt.

Ein so hergestellter erfindungsgemäßer Katalysator kann direkt zur selektiven Hydrierung eingesetzt werden.

Die Erfindung wird durch die folgenden Beispiele näher erläutert:

Als Beispiel für eine selektive Hydrierung wird die Reaktion von α-Melhylstyrol zu Cumol verwendet. Als unerwünschtes Nebenprodukt kann hier das kernhydrierte Isopropylcyclohexan auftreten, dessen Bildung erheblichen negativen Einfluß auf die Reaktion und damit auf die Ausbeute an Cumol hat.

Beispiel 1

Der erfindungsgemäße Katalysator 1 wird wie folgt hergestellt: Als Aluminiumoxidvorläuferverbindung für den Aluminiumoxidträger verwendet man Bayerit, der im Mischer mit Salpetersäure behandelt und anschließend verformt wird. Der Formkörper wird dann bei einer Temperatur von 120 °C getrocknet und bei einer Temperatur von 650 °C kalziniert.

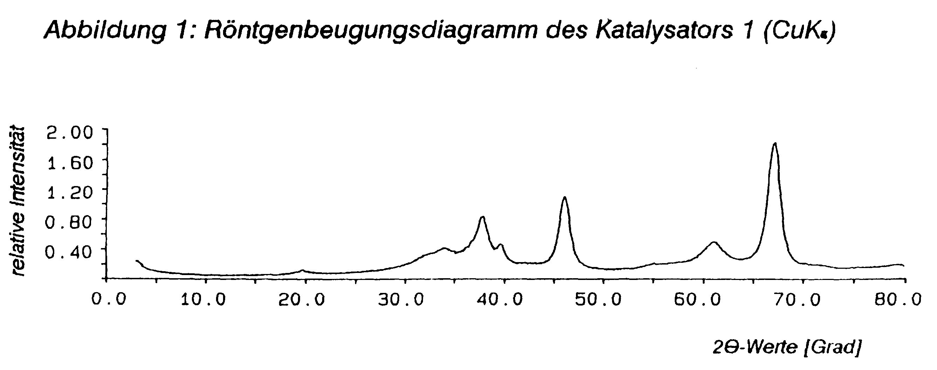

Den so erhaltenen Aluminiumoxidträger imprägniert man mit einer wäßrigen Palladium(II)nitrat-Lösung, wobei die Temperatur des Aluminiumoxidträgers ca. 25 °C beträgt. Der imprägnierte Aluminiumoxidträger wird nun bei einer Temperatur von 110 °C getrocknet und anschließend bei einer Temperatur von 450 °C kalziniert. Der so hergestellte Katalysator 1 enthält 0,5 Gew.-% Palladium sowie Palladiumoxid, gerechnet als Pd. Abb. 1 zeigt das Röntgenbeugungsdiagramm (Cu K$\overline{\alpha}$) des Katalysators 1. Es sind die relativen Intensitäten in Abhängigkeit der Beugungswinkel, angegeben in 2 θ-Werten [Grad], aufgetragen.

20 g des Katalysators 1 werden in einer Flüssigphasenkreislaufapparatur, die aus Reaktor, Wärmetauscher, Abscheider und Pumpe besteht, vorgelegt.

Die Flüssigphasenkreislaufapparatur wird mit 1 000 cm$^3$ technischem, flüssigem Einsatzstoff nachstehender Zusammensetzung (siehe Tabelle 1) gefüllt, verschlossen, mit Stickstoff gespült und auf eine Reaktionstemperatur von 70 °C aufgeheizt. Anschließend wird der Einsatzstoff bei einem Wasserstoffdruck von 8 bar abs. hydriert. Die chemischen Zusammensetzungen des Einsatzstoffes und Hydrierproduktes sind in Tabelle 1 wiedergegeben (siehe unten).

Nach dreistündiger Hydrierung beträgt der Restgehalt an α-Methylstyrol im Hydrierprodukt weniger als 10 Gew.-ppm (siehe Tabelle 1). Der Gehalt an Isopropylcyclohexan ist nur um 90 Gew.-ppm angestiegen.

Tabelle 1

|  | Einsatzstoff | Hydrierprodukt |
|---|---|---|
| Cumol | 73,3 Gew.-% | 91,2 Gew.-% |
| α-Methylstyrol | 18,1 Gew.-% | <0,001 Gew.-% |
| Isopropylcyclohexan | 0,005 Gew.-% | 0,014 Gew.-% |

Beispiel 2

Der erfindungsgemäße Katalysator 2 wird wie folgt hergestellt: Als Aluminiumoxidvorläuferverbindung für den Aluminiumoxidträger verwendet man Pseudo-Boehmit, der im Kneter mit Salpetersäure behandelt und anschließend verformt wird. Der Formkörper wird dann bei einer Temperatur von 120 °C getrocknet und bei einer Temperatur von 550 °C kalziniert.

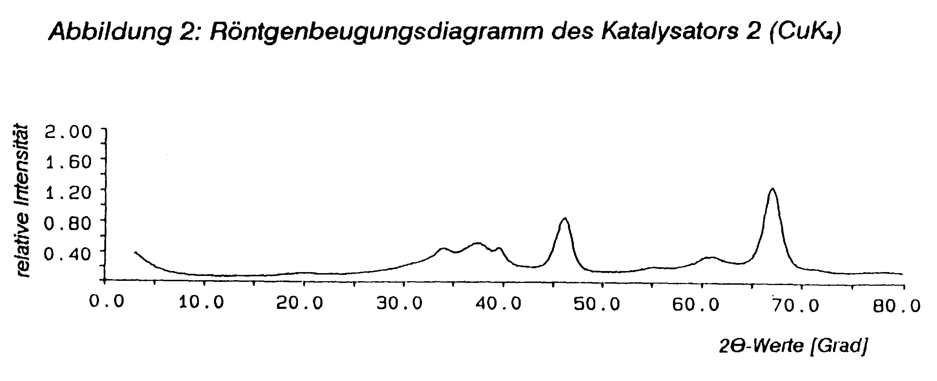

Den so erhaltenen Aluminiumoxidträger imprägniert man mit einer wäßrigen Palladium(II)nitrat-Lösung, wobei die Temperatur des Aluminiumoxidträgers 95 - 100 °C beträgt. Der imprägnierte Aluminiumoxidträger wird nun bei einer Temperatur von 130 °C getrocknet und anschließend bei einer Temperatur von 450 °C kalziniert. Der so hergestellte Katalysator 2 enthält 1 Gew.-% Palladium sowie Palladiumoxid, gerechnet als Pd. Abb. 2 zeigt das Röntgenbeugungsdiagramm (Cu K$\overline{\alpha}$) des Katalysators 2. Es sind die relativen Intensitäten in Abhängigkeit der Beugungswinkel, angegeben in 2 θ-Werten (Grad], aufgetragen.

Der Einsatzstoff gemäß Tabelle 2 (siehe unten) wird mit Hilfe des Katalysators 2 wie im Beispiel 1 hydriert, jedoch beträgt die Reaktionstemperatur 110 °C.

Die chemischen Zusammensetzungen des Einsatzstoffes und Hydrierproduktes sind in Tabelle 2 wiedergegeben (siehe unten).

Tabelle 2

|  | Einsatzstoff | Hydrierprodukt |
|---|---|---|
| Cumol | 73,6 Gew.-% | 91,6 Gew.-% |
| α-Methylstyrol | 18,0 Gew.-% | 0,029 Gew.-% |
| Isopropylcyclohexan | 0,005 Gew.-% | 0,016 Gew.-% |

Nach zweistündiger Reaktionszeit beträgt der Gehalt an α-Methylstyrol im Hydrierprodukt nur noch 0,029 Gew.-%. Der Gehalt an Isopropylcyclohexan ist nur um 110 Gew.-ppm angestiegen.

Beispiel 3

Die selektive Hydrierung wird gemäß Beispiel 1 durchgeführt, jedoch beträgt die Reaktionstemperatur 130 °C. Als Hydrierungskatalysator wird der Katalysator 1 aus Beispiel 1 verwendet. Ausgehend von 18 Gew.-% α-Methylstyrol im Einsatzstoff ergibt sich nach 2 Stunden Reaktionszeit ein Restgehalt von weniger als 10 Gew.-ppm α-Methylstyrol im Hydrierprodukt. Der Gehalt an Isopropylcyclohexan ist nur um 160 Gew.-ppm angestiegen.

**Patentansprüche**

1. Katalysator zur selektiven Hydrierung ungesättigter Verbindungen auf Basis von Edelmetall und/oder Edelmetalloxid auf einem Aluminiumoxidträger,
dadurch gekennzeichnet,
daß der Frischkatalysator das folgende Röntgenbeugungsmuster aufweist:

| d[$10^{-10}$m] | I/Io |
|---|---|
| 4,51 | 5-10 |
| 2,76 | 15-40 |
| 2,64 |  |
| 2,37 | 40-45 |
| 2,27 | 25-35 |
| 2,15 | bis 15 |
| 1,97 | 55-70 |
| 1,66 | bis 15 |
| 1,52 | 15-30 |
| 1,39 | 100 |

(fortgesetzt)

| $d[10^{-10}m]$ | I/Io |
|---|---|
| 1,31 | bis 15 |

**2.** Katalysator nach Anspruch 1,
dadurch gekennzeichnet
daß der Katalysator Palladium und/oder Palladiumoxid der Formel $PdO_x$ mit x = 0 bis 1 enthält.

**3.** Katalysator nach den Ansprüchen 1 und 2,
dadurch gekennzeichnet,
daß der Katalysator nein Palladium und/oder Palladiumoxid Edelmetalle und/oder Edelmetalloxide der Gruppe Platin, Iridium, Ruthenium und Rhodium enthält.

**4.** Katalysator nach den Ansprüchen 1 bis 3,
dadurch gekennzeichnet,
daß der Gehalt an Edelmetallen und/oder Edellmetalloxiden insgesamt 0,01 bis 3 Gew.-%. bezogen auf den Katalysator, beträgt.

**5.** Katalysator nach Anspruch 4,
dadurch gekennzeichnet,
daß der Gehalt an Edelmetallen und/oder Edelmetalloxiden insgesamt 0,1 bis 1 Gew.-%. bezogen auf den Katalysator, beträgt.

**6.** Katalysator nach den Ansprüchen 1 bis 5,
dadurch gekennzeichnet,
daß die Edelmetalle und/oder Edelmetalloxide sich in einer äußeren Randzone des Katalysators im Bereich von 5 bis 200 μm befinden.

**7.** Katalysator nach Anspruch 6,
dadurch gekennzeichnet,
daß die Edelmetalle und/oder Edelmetalloxide sich in einer äußeren Randzone des Katalysators im Bereich von 30 bis 100 μm befinden.

**8.** Katalysator nach den Ansprüchen 1 bis 7,
dadurch gekennzeichnet,
daß die im Frischkatalysator enthaltenen Primärkristallite eine Größe von 0,1 bis 30 nm aufweisen.

**9.** Katalysator nach Anspruch 8,
dadurch gekennzeichnet,
daß die im Frischkatalysator enthaltenen Primärkristallite eine Größe von 1 bis 15 nm aufweisen.

**10.** Katalysator nach den Ansprüchen 1 bis 9,
dadurch gekennzeichnet.
daß der Natriumgehalt des Frischkatalysators 0,001 bis 3 Gew.-% beträgt.

**11.** Katalysator nach Anspruch 10,
dadurch gekennzeichnet,
daß der Natriumgehalt des Frischkatalysators 0,01 bis 1 Gew.-% beträgt.

**12.** Katalysator nach den Ansprüchen 1 bis 11,
erhältlich durch das Einbringen einer wäßrigen Edelmetallsalzlösung in den Aluminiumoxidträger.

**13.** Katalysator nach Anspruch 12,
erhältlich durch Trocknen des mit einer wäßrigen Edelmetallsalzlösung behandelten Aluminiumoxidträgers bei einer Temperatur von 50 bis 200 °C und anschließendes thermisches Nachbehandeln bei einer Temperatur von 250 bis 650 °C.

**14.** Verfahren zur selektiven Hydrierung ungesättigter Verbindungen, dadurch gekennzeichnet, daß ein Katalysator gemäß den Ansprüchen 1 bis 11 eingesetzt wird.

**15.** Verfahren nach Anspruch 14, dadurch gekennzeichnet, daß die selektive Hydrierung bei Drücken von 2 bis 30 bar abs, und bei Temperaturen von 40 bis 180 °C durchge führt wird.

**16.** Verfahren nach den Ansprüchen 14 und 15, dadurch gekennzeichnet, daß ein Molverhältnis von Wasserstoff zu ungesättigten Verbindungen von 1 : 1 bis 3 : 1 eingestellt wird.

**17.** Verfahren nach den Ansprüchen 14 und 16, dadurch gekennzeichnet, daß als ungesättigte Verbindung α-Methylstyrol eingesetzt wird.

**Claims**

**1.** A catalyst for the selective hydrogenation of unsaturated compounds based on a noble metal and/or a noble-metal oxide on an aluminium oxide support, characterised in that the fresh catalyst has the following X-ray diffraction pattern:

| d[$10^{-10}$m] | l/lo |
|---|---|
| 4.51 | 5-10 |
| 2.76 | 15-40 |
| 2.64 | |
| 2.37 | 40-45 |
| 2.27 | 25-35 |
| 2.15 | up to 15 |
| 1.97 | 55-70 |
| 1.66 | up to 15 |
| 1.52 | 15-30 |
| 1.39 | 100 |
| 1.31 | up to 15 |

**2.** A catalyst according to claim 1, characterised in that the catalyst contains palladium and/or palladium oxide of the formula $PdO_x$ where x = 0 to 1.

**3.** A catalyst according to either of claims 1 and 2, characterised in that the catalyst contains, in addition to palladium and/or palladium oxide, noble metals and/or noble-metal oxides of the group consisting of platinum, iridium, ruthenium and rhodium.

**4.** A catalyst according to any of claims 1 to 3, characterised in that the content of noble metals and/or noble-metal oxides is in total from 0.01 to 3% by weight, based on the catalyst.

**5.** A catalyst according to claim 4, characterised in that the content of noble metals and/or noble-metal oxides is in total from 0.1 to 1% by weight, based on the catalyst.

**6.** A catalyst according to any of claims 1 to 5, characterised in that the noble metals and/or noble-metal oxides are present in an outer peripheral zone of the catalyst in the range from 5 to 200 μm.

**7.** A catalyst according to claim 6, characterised in that the noble metals and/or noble-metal oxides are present in an outer peripheral zone of the catalyst in the range from 30 to 100 μm.

**8.** A catalyst according to any of claims 1 to 7, characterised in that the primary crystallites present in the fresh catalyst have a size of from 0.1 to 30 nm.

**9.** A catalyst according to claim 8, characterised in that the primary crystallites in the fresh catalyst have a size of from 1 to 15 nm.

**10.** A catalyst according to any of claims 1 to 9, characterised in that the sodium content of the fresh catalyst is from 0.001 to 3% by weight.

**11.** A catalyst according to claim 10, characterised in that the sodium content of the fresh catalyst is from 0.01 to 1% by weight.

**12.** A catalyst according to any of claims 1 to 11, obtainable by introducing an aqueous noble-metal salt solution into the aluminium oxide support.

**13.** A catalyst according to claim 12, obtainable by subjecting the aluminium oxide support which has been treated with an aqueous noble-metal salt solution to drying at a temperature of from 50 to 200°C and subsequently to heating at a temperature of from 250 to 650°C.

**14.** A process for the selective hydrogenation of unsaturated compounds, characterised in that a catalyst according to any of claims 1 to 11 is employed.

**15.** A process according to claim 14, characterised in that the selective hydrogenation is carried out at pressures of from 2 to 30 bar abs. and at temperatures of from 40 to 180°C.

**16.** A process according to either of claims 14 and 15, characterised in that a molar ratio between hydrogen and unsaturated compounds of from 1:1 to 3:1 is set.

**17.** A process according to any of claims 14 to 16, characterised in that α-methylstyrene is employed as unsaturated compound.

**Revendications**

**1.** Catalyseur pour l'hydrogénation sélective de composés non saturés à base de métal noble et/au d'oxyde de métal noble sur un support en oxyde d'aluminium,
caractérisé en ce que
le catalyseur frais possède le modèle suivant de diffraction aux rayons X :

EP 0 576 828 B2

| d [$10^{-10}$m] | I/Io |
|---|---|
| 4,51 | 5 - 10 |
| 2,76 } | 15 - 40 |
| 2,64 } | |
| 2,37 | 40 - 45 |
| 2,27 | 25 - 35 |
| 2,15 | jusqu'à 15 |
| 1,97 | 55 - 70 |
| 1,66 | jusqu'à 15 |
| 1,52 | 15 - 30 |
| 1,39 | 100 |
| 1,31 | jusqu'à 15 |

**2.** Catalyseur selon la revendication 1,
caractérisé en ce que
le catalyseur renferme du palladium et/au de oxyde de palladium de formule $PdO_x$ avec x = 0 à 1.

**3.** Catalyseur selon les revendications 1 et 2,
caractérisé en ce que
le catalyseur renferme à côté du palladium et/ou de l'oxyde de palladium, des métaux nobles et/ou des oxydes de métal noble, du groupe du platine, de l'iridium, du ruthénium et du rhodium.

**4.** Catalyseur selon les revendications 1 à 3,
caractérisé en ce que
la teneur en métaux nobles et/ou en oxydes de métal noble s'élève au total de 0,01 à 3 % en poids, rapporté au catalyseur.

**5.** Catalyseur selon la revendication 4,
caractérisé en ce que
la teneur en métaux nobles et/ou en oxydes de métal noble s'élève au total de 0,1 à 1 % en poids, rapporté au catalyseur.

**6.** Catalyseur selon les revendications 1 à 5,
caractérisé en ce que
les métaux nobles et/ou les oxydes de métal noble se trouvent dans la zone extérieure périphérique du catalyseur dans la zone de 5 à 200 μm.

**7.** Catalyseur selon la revendication 6,
caractérisé en ce que
les métaux nobles et/ou les oxydes de métal noble se trouvent dans la zone extérieure périphérique du catalyseur dans la zone de 30 à 100 μm.

8. Catalyseur selon les revendications 1 à 7,
   caractérisé en ce que
   les cristallites primaires contenus dans le catalyseur frais, possèdent une taille allant de 0,1 à 30 nm.

9. Catalyseur selon la revendication 8,
   caractérisé en ce que
   les cristallites primaires contenus dans le catalyseur frais possèdent une taille allant de 1 à 15 nm.

10. Catalyseur selon les revendications 1 à 9,
    caractérisé en ce que
    la teneur en sodium du catalyseur frais s'élève de 0,001 à 3 % en poids.

11. Catalyseur selon la revendication 10,
    caractérisé en ce que
    la teneur en sodium du catalyseur frais s'élève de 0,01 à 1 % en poids.

12. Catalyseur selon les revendications 1 à 11, que l'on peut obtenir par l'inclusion d'une solution de sel de métal noble aqueuse dans le support d'oxyde d'aluminium.

13. Catalyseur selon la revendication 12, que l'on peut obtenir par séchage d'un support en oxyde d'aluminium traité par une solution aqueuse de sel de métal noble à une température de 50 à 200°C et ensuite à un traitement à une température de 250° à 650°C.

14. Procédé d'hydrogénation sélective de composés non saturés,
    caracterisé en ce que
    l'on met en oeuvre un catalyseur selon les revendications 1 à 11,

15. Procédé selon la revendication 14,
    caractérisé en ce que
    l'hydrogénation catalytique est effectuée à des pressions allant de 2 à 30 bars (abs) et à des températures allant de 40 à 180°C.

16. Procédé selon les revendications 14 à 15,
    caractérisé en ce que
    l'on ajuste un rapport molaire d'hydrogène aux composés non saturés de 1:1 à 3:1.

17. Procédé selon les revendications 14 à 16,
    caractérisé en ce que
    l'on met en oeuvre comme composé non saturé un α-méthylstyrène.

11

*Abbildung 1: Röntgenbeugungsdiagramm des Katalysators 1 (CuKₐ)*

relative Intensität

2.00
1.60
1.20
0.80
0.40

0.0    10.0    20.0    30.0    40.0    50.0    60.0    70.0    80.0

*2Θ-Werte [Grad]*

EP 0 576 828 B2

Abbildung 2: Röntgenbeugungsdiagramm des Katalysators 2 (CuKₐ)